(19) Europäisches Patentamt
European Patent Office
Office européen des brevets

(11) **EP 4 086 664 A1**

(12) **EUROPEAN PATENT APPLICATION**
published in accordance with Art. 153(4) EPC

(43) Date of publication:
**09.11.2022 Bulletin 2022/45**

(21) Application number: **20910426.4**

(22) Date of filing: **03.09.2020**

(51) International Patent Classification (IPC):
**G01T 1/17** [(2006.01)]

(86) International application number:
**PCT/CN2020/113208**

(87) International publication number:
**WO 2021/135338 (08.07.2021 Gazette 2021/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.01.2020 CN 202010000182**

(71) Applicant: **Raycan Technology Co., Ltd. (Suzhou) Suzhou New District**
**Suzhou, Jiangsu 215163 (CN)**

(72) Inventors:
• **XIE, Qingguo**
**Suzhou, Jiangsu 215163 (CN)**
• **SU, Yuming**
**Suzhou, Jiangsu 215163 (CN)**
• **DAI, Pingping**
**Suzhou, Jiangsu 215163 (CN)**
• **MEI, Junhua**
**Suzhou, Jiangsu 215163 (CN)**
• **WAN, Lin**
**Suzhou, Jiangsu 215163 (CN)**
• **ZHU, Kezhang**
**Suzhou, Jiangsu 215163 (CN)**

(74) Representative: **Müller Verweyen**
**Patentanwälte**
**Friedensallee 290**
**22763 Hamburg (DE)**

(54) **SIGNAL SAMPLING AND RECONSTRUCTION METHOD AND DEVICE**

(57)      Disclosed in the embodiments of the application are signal sampling and reconstruction methods and devices. This signal sampling method comprises: sampling an electrical signal to be measured using a pre-determined sampling method to obtain a plurality of first sampling points, each of which is represented by a first amplitude and a corresponding first time; measuring a second amplitude of the electrical signal to be measured, wherein the second amplitude is different from the plurality of the first amplitudes; and delaying the electrical signal to be measured, and using the delayed electrical signal to determine a second time when the amplitude of the electrical signal to be measured reaches the second amplitude in order to obtain a second sampling point, which is represented by the second amplitude and the second time. By means of the technical solution provided in the embodiments of the application, a greater number of sampling points can be sampled, such that the precision of sampling and also the accuracy of subsequent signal restoration may be improved.

Sampling an electrical signal to be measured using a pre-determined sampling method to obtain a plurality of first sampling points — S1

Measuring a second amplitude of the electrical signal to be measured — S2

Delaying the electrical signal to be measured, and using the delayed electrical signal to determine a second time when the amplitude of the electrical signal to be measured reaches the second amplitude in order to obtain a second sampling point — S3

FIG. 1

## Description

The application claims the priority of Chinese patent application 202010000182.8 filed on January 2, 2020, the entire contents of which are incorporated herein by reference.

## TECHNICAL FIELD

The application relates to the technical field of signal processing, and in particular to signal sampling and reconstruction methods and devices.

## BACKGROUND

Positron Emission Tomography (PET) is a technique that adopts radioactive elements for clinical imaging by labeling positron-emitting radionuclides on compounds that may participate in the blood flow or metabolism of living tissues and then injecting the compounds labeled with radionuclides into the subject. The positron emitted by the radionuclide in the body combines with the negative electron in the subject to annihilate the electron pair and produce gamma photons, and the gamma photons released may be received by the scintillation crystal and converted into visible light, and then converted into electrical signals by the photomultiplier element for reconstruction, thereby helping determine the enrichment site of radionuclides, and helping locate the area of vigorous metabolism and assess activity.

In traditional techniques, an analog-to-digital converter (ADC) is usually used to digitally sample the electrical signals generated by the PET detectors. However, the use of the ADC to digitally sample the electrical signals is difficult to implement with an extremely expensive cost.

In the field of radiation detection and imaging, the multi-voltage threshold (MVT) sampling method is typically used in the prior art, in order to reduce the cost of digital sampling of electrical signals. However, only a limited number of the sampling points may be sampled using the method, and it is incapable of determining whether the sample points sampled are those of signal extreme amplitude value points, which may affect the precision of sampling and also the accuracy of subsequent signal restoration.

## SUMMARY

The object of the embodiments of the present application is to provide signal sampling and reconstruction methods and devices to solve at least one technical problem in the prior art.

In order to solve the above technical problems, provided in embodiments of the present application is a signal sampling method, comprising:

sampling an electrical signal to be measured using a pre-determined sampling method to obtain a plurality of first sampling points, each of which is represented by a first amplitude and a corresponding first time;

measuring a second amplitude of the electrical signal to be measured, wherein the second amplitude is different from the plurality of the first amplitudes; and

delaying the electrical signal to be measured, and using the delayed electrical signal to determine a second time when the amplitude of the electrical signal to be measured reaches the second amplitude in order to obtain a second sampling point, which is represented by the second amplitude and the second time.

Optionally, the step of determining the second time comprises:

measuring a first delay time and a second delay time when the amplitude of the delayed electrical signal reaches any of the first amplitudes and the second amplitude, respectively;

calculating a difference between the first delay time and the second delay time; and

calculating the second time corresponding to the second amplitude using the difference and the first time corresponding to the first amplitude.

Optionally, the pre-determined sampling method comprises a multi-amplitude threshold sampling method or a time interval sampling method.

Optionally, the electrical signal to be measured comprises one of the following types of pulse signals generated by a PET detector: a sine wave signal, a cosine wave signal, a triangle wave signal, a sawtooth wave signal, a step wave signal, or a square wave signal.

Optionally, the second amplitude comprises a maximum or minimum amplitude of the electrical signal to be measured.

Further provided in the embodiments of the present application is a signal reconstruction method, comprising:

conducting a reconstruction with the first sampling points and the second sampling point of the electrical signal to be measured which are sampled by means of the above-mentioned signal sampling method to obtain a reconstructed waveform of the electrical signal to be measured.

Optionally, the step of conducting a reconstruction with the first sampling points and the second sampling point comprises:

conducting a fitting to the first sampling points and the second sampling point;

conducting an interpolation of the first sampling points and the second sampling point; or

conducting an interpolation of the first sampling points and the second sampling point, and a fitting to all the sampling points after interpolation, wherein the interpolation comprises a linear interpolation and/or a spline interpolation.

Further provided in embodiments of the present application is a signal sampling device, comprising:

a first sampling unit configured to sample an electrical signal to be measured using a pre-determined sampling method to obtain a plurality of first sampling points, each of which is represented by a first amplitude and a corresponding first time;

a measuring unit configured to measure a second amplitude of the electrical signal to be measured, wherein the second amplitude is different from the plurality of the first amplitudes; and

a second sampling unit configured to delay the electrical signal to be measured, and use the delayed electrical signal to determine a second time when the amplitude of the electrical signal to be measured reaches the second amplitude in order to obtain a second sampling point, which is represented by the second amplitude and the second time.

Optionally, the measuring unit comprises a voltage retaining circuit consisting of a capacitor, a diode and an inductor, wherein one end of the capacitor and one end of the inductor are connected in parallel and grounded, the other end of the capacitor and one end of the diode are connected in parallel, and the other end of the diode and the other end of the inductor are connected in series.

Further provided in embodiments of the present application is a signal reconstruction device used for conducting a reconstruction with the first sampling points and the second sampling point sampled by the above-mentioned signal sampling device to obtain a reconstructed waveform of the electrical signal to be measured.

As can be seen from the above-mentioned technical solution provided in the embodiments of the application, the embodiments of the present propose sampling an electrical signal to be measured using a pre-determined sampling method to obtain a plurality of first sampling points, measuring a second amplitude of the electrical signal to be measured, delaying the electrical signal to be measured and using the delayed electrical signal to determine a second time when the amplitude of the electrical signal to be measured reaches the second amplitude in order to obtain a second sampling point, which increases the number of the sampling points as sampled, such that the precision of sampling and the accuracy of subsequent signal restoration may be improved.

BRIEF DESCRIPTION OF THE DRAWINGS

The accompanying drawings with reference to the description of the embodiments or the prior art will be briefly described for the purpose of demonstrating the technical solutions of the embodiments in the disclosure or the prior art. It is apparent that the drawings as shown are merely illustrative of some embodiments as recited in the disclosure. It should be understood by those skilled in the art that various alternatives to the drawings as shown may be appreciated, without creative work involved.

Fig. 1 is a flowchart of the signal sampling method according to an embodiment of the application;
Fig. 2 is a schematically structural diagram of the voltage retaining circuit;
Fig. 3 is a schematic diagram of the waveform of the electrical signal to be measured;
Fig. 4 is a schematic diagram of the waveform of the electrical signal output by the voltage retaining circuit for the electrical signal to be measured in Fig. 3; and
Fig 5 is a schematically structural diagram of a signal sampling device provided in an embodiment of the application.

DETAILED DESCRIPTION OF EMBODIMENTS

In the following, the technical solutions in the embodiments of the present application will be clearly and completely described in conjunction with the accompanying drawings of the embodiments of the present application. Obviously, the described embodiments are only parts of the embodiments of the application for construing the application, not all of them, and are not intended to limit the scope of the application or the claims. It should be understood that various alternatives to the embodiments described herein may be employed by those skilled in the art without creative work involved and without departing from the spirit and scope of the invention.

Notably, when an element is referred to as being "disposed on" another element, it can be directly disposed on another element or there may be an intermediate element. When an element is referred to as being "connected or coupled" to another element, it may be directly connected or coupled to the other element or there is an intermediate element. The term "connection or coupling" used herein may include electrical connection or coupling and/or mechanical or physical connection or coupling. The term "comprise or include" used herein refers to the existence of features, steps or elements, but does not exclude the existence or addition of one or more further features, steps or elements. The term "and/or" used herein includes any and all combinations of one or more of the related listed items.

Unless otherwise indicated, all the technical and scientific terms used herein have general meaning as commonly understood by those skilled in the technical field related to the disclosure. The terms used herein are for the purpose of describing specific embodiments, but not intended to limit the invention.

In addition, the terms "first", "second", "third" or the like used herein are only for the purpose of description and to distinguish similar objects from each other, which do not express the sequence thereof, nor can they be understood as indication or implication of relative importance. In addition, in the description in the disclosure, unless otherwise specified, "a plurality of" means two or more.

Hereinafter, a signal sampling method, a reconstruction method, and devices thereof according to the embodiments of the application will be described in detail with reference to the accompanying drawings.

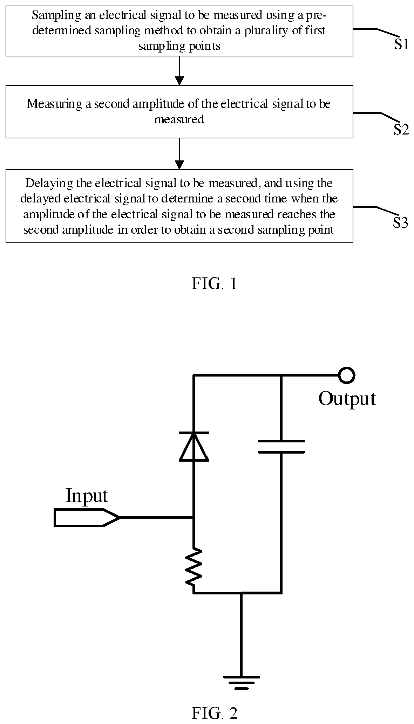

As shown in Fig. 1, an embodiment of the present application provides a signal sampling method, which may comprise the following steps of:

S1: sampling an electrical signal to be measured using a pre-determined sampling method to obtain a plurality of first sampling points.

The electrical signal to be measured, after being obtained, may be sampled using the pre-determined sampling method, such as multi-amplitude threshold sampling (e.g., MVT) method or time interval sampling method to obtain the plurality of the first sampling points. For example, the electrical signal to be measured may be sampled with a plurality of pre-set first amplitudes, to determine the first times when the amplitude of the electrical signal to be measured reaches the first amplitudes, such that the plurality of first sampling points may be obtained. For example, the electrical signal to be measured may be sampled by a pre-set time interval, to determine the first amplitudes corresponding to a plurality of first times corresponding to the pre-set time interval, such that the plurality of first sampling points may also be obtained. Each of the first sampling points may be represented by one first amplitude and one corresponding first time, such as (T1, V1), (T2, V2), (T3, V2), or (T4, VI).

It is noted that the plurality of first amplitudes may be different from each other, and also the plurality of first times may be different from each other. Furthermore, in the multi-amplitude threshold sampling method the first amplitudes may be pre-set amplitude thresholds, while the first times may be the times as measured corresponding to the amplitude thresholds; while in the time interval sampling method, the first amplitudes may be the measured amplitudes of the electrical signal to be measured, and the first times may be the times calculated according to the pre-set time interval. In general, one first amplitude corresponds to two first times.

It may refer to the relevant description in the prior arts on how to sample the electrical signal using the multi-amplitude threshold sampling method and the time interval sampling method, which will not be further elaborated herein.

The electrical signal to be measured may be a pulse signal generated by a PET detector, e.g., a periodic signal, such as a sine wave signal, a cosine wave signal, a triangle wave signal, a sawtooth wave signal, a step wave signal, or a square wave signal, or other electrical signals which need to be measured and will not be limited herein.

S2: measuring a second amplitude of the electrical signal to be measured.

After the electrical signal to be measured is obtained, a second amplitude of the electrical signal to be measured may be measured using a voltage retaining circuit. The second amplitude may be different from the plurality of the first amplitudes. Further, the second amplitude may be any amplitude different from the first amplitudes, preferably the maximum amplitude of the electrical signal to be measured. Further, if the electrical signal to be measured is a signal of which the amplitude decreases first and then increases, e.g., a cosine wave signal, the second amplitude may also be the minimum amplitude.

The voltage retaining circuit may be designed in advance according to actual needs or experiences, which may hold the voltage of the output thereof unchanged when the amplitude of the electrical signal to be measured reaches the internally set amplitude threshold thereof, or may hold the voltage of the output thereof when the amplitude of the electrical signal to be measured reaches a peak value i.e., the maximum amplitude or minimum amplitude of the electrical signal to be measured. By means of the pre-designed voltage retaining circuit, the second amplitude of the electrical signal to be measured may be different from the first amplitudes.

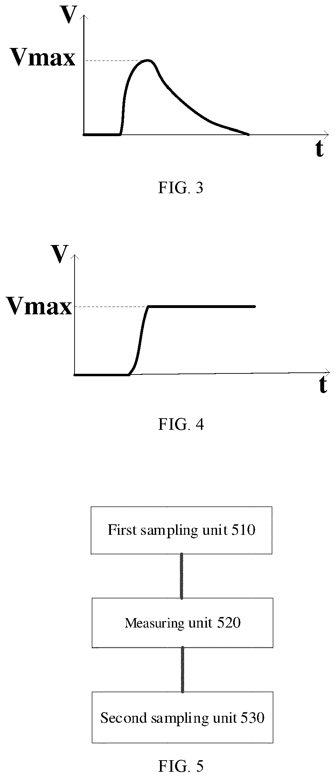

In the case that the second amplitude is the maximum amplitude of the electrical signal to be measured, the voltage retaining circuit may comprise a capacitor, a diode, an inductor, and the like component. One end of the capacitor and one end of the inductor are connected in parallel and grounded, the other end of the capacitor and one end of the diode are connected in parallel, and the other end of the diode and the other end of the inductor are connected in series, as shown in Fig. 2. The diode may be conducted before the amplitude of the electrical signal to be measured reaches the peak value, and be disconnected when the amplitude of the electrical signal to be measured reaches the peak value, such that the voltage of the capacitor may stay stabilized for a period of time. During the measuring, when the voltage across the capacitor suddenly changes, for example, by jumping from Vi to Vj where i and j are different positive integers, the current amplitude of the electrical signal to be measured is recorded as the second amplitude. For example, for the electrical signal to be measured as shown in Fig. 3, the electrical signal output by the voltage retaining circuit is shown in Fig. 4, where Vmax is the second amplitude as measured.

When the second amplitude is the minimum amplitude of the electrical signal to be measured, the voltage retaining circuit may also be of other components or circuit structures for holding the voltage, and will not be limited herein.

It shall be noted that in the disclosure there is no limitation to the execution order of the step S1 and step S2, such that the step S1 may also be executed after the execution of the step S2.

S3: delaying the electrical signal to be measured, and using the delayed electrical signal to determine a second time when the amplitude of the electrical signal to be measured reaches the second amplitude in order to obtain a second sampling point.

The electrical signal to be measured, after being obtained, may be delayed, e.g., by 30ns. It may refer to the relevant description in the prior arts on how to delay the electrical signal, which will not be further elaborated herein.

It shall be noted that in the disclosure there is no limitation to the execution order of measuring a second amplitude of the electrical signal to be measured and delaying the electrical signal to be measured, such that the two may be executed sequentially or simultaneously.

After delaying the electrical signal to be measured, it may use the delayed electrical signal to determine a second time when the amplitude of the electrical signal to be measured reaches the second amplitude in order to obtain a second sampling point, which is represented by the second amplitude and the second time. If the second amplitude is the maximum or minimum amplitude, the second sampling point as sampled will be the extreme amplitude value point (i.e., peak or trough) of the electrical signal to be measured.

Using the delayed electrical signal to determine a second time when the amplitude of the electrical signal to be measured reaches the second amplitude, may comprise: first, measuring a first delay time and a second delay time when the amplitude of the delayed electrical signal reaches any of the first amplitudes and the second amplitude, respectively; second, calculating a difference between the first delay time and the second delay time; and finally calculating the second time corresponding to the second amplitude using the difference and the first time corresponding to the first amplitude. The calculation process may be expressed in the following equation:

$$T_j = T_i + \Delta T = T_i + (T_j' - T_i') .$$

wherein $T_j$ represents the second time, $T_i$ represents the first time, which may be either of the two first times corresponding to the selected first amplitude, $T_j'$ represents the second delay time, and $T_i'$ may be the first delay time corresponding to the first time $T_i$.

In the foregoing embodiment, the first amplitudes and the second amplitude may be of voltage, current or the like, and may also be other physical quantities for expressing the amplitude.

As can be seen from the above description, the embodiments of the present application propose sampling an electrical signal to be measured using a pre-determined sampling method to obtain a plurality of first sampling points, measuring a second amplitude of the electrical signal to be measured, delaying the electrical signal to be measured and using the delayed electrical signal to determine a second time when the amplitude of the electrical signal to be measured reaches the second amplitude in order to obtain a second sampling point, which increases the number of the sampling points as sampled, such that the precision of subsequent signal restoration may be improved. By means of the technical solution of the application, moreover, it may self-adaptively sample the amplitude value point (i.e., peaks or troughs) of the electrical signal, which facilitates to improve the precision of the subsequent signal restoration, thereby increasing the energy resolution.

Further provided in the embodiments of the present application is a signal reconstruction method, comprising conducting a reconstruction with the first sampling points and the second sampling point of the electrical signal to be measured which are sampled by means of the signal sampling method described in the above embodiments.

The first sampling points and the second sampling point of the electrical signal, after being obtained, may be used for conducting the reconstruction to obtain a reconstructed waveform of the electrical signal. This step may specifically comprise : conducting a fitting directly to the first sampling points and the second sampling point using a priori model or a characteristic function of the electrical signal (e.g., y(t)=a*exp(-(t-d)/b)*(1-exp(-(t-d)/c))); or alternatively, conducting an interpolation of the first sampling points and the second sampling point directly; or alternatively, conducting an interpolation of the first sampling points and the second sampling point to obtain more sampling points, and then a fitting to the sampling points after interpolation. The interpolation may comprise, but is not limited to, a linear interpolation and/or a spline interpolation. It may refer to the relevant description in the prior arts on the specific process of the interpolation of the sampling points, which will not be further elaborated herein.

By means of the signal reconstruction method provided in the embodiments of the application, the precision of the signal restoration can be improved.

Further provided in embodiments of the present application is a signal sampling device as shown in Fig. 5, comprising:

a first sampling unit 510 used for sampling an electrical signal to be measured using a pre-determined sampling method to obtain a plurality of first sampling points, each of which is represented by a first amplitude and a corresponding first time;

a measuring unit 520 used for measuring a second amplitude of the electrical signal to be measured, wherein the second amplitude is different from the plurality of the first amplitudes;

a second sampling unit 530 used for delaying the electrical signal to be measured, and using the delayed electrical signal to determine a second time when the amplitude of the electrical signal to be measured reaches the second amplitude in order to obtain a second sampling point, which is represented by the second amplitude and the second time.

In an embodiment, the measuring unit 520 comprises a voltage retaining circuit consisting of a capacitor, a diode and an inductor. One end of the capacitor and one end of the inductor are connected in parallel and grounded, the other end of the capacitor and one end of the diode are connected in parallel, and the other end of the diode and the other end of the inductor are connected in series. The voltage retaining circuit may measure the maximum amplitude of the electrical signal to be measured.

In an embodiment, the second sampling unit 530 may specifically include (not shown):

a measuring subunit used for measuring a first delay time and a second delay time when the amplitude of the delayed electrical signal reaches any of the first amplitudes and the second amplitude, respectively; and
a calculating subunit used for calculating a difference between the first delay time and the second delay time and then calculating the second time corresponding to the second amplitude using the difference and the first time corresponding to the first amplitude.

Reference may be made to the detailed description of the signal sampling method in the foregoing embodiments for the description of the signal sampling device, which will not be elaborated herein.

As can be seen from the above description, the signal sampling device provided in the embodiments of the present application may use a first sampling unit to sample an electrical signal to be measured using a pre-determined sampling method to obtain a plurality of first sampling points, use a measuring unit to measure a second amplitude of the electrical signal to be measured, use a delay unit to delay the electrical signal to be measured and use a second sampling unit, based on the delayed electrical signal, to determine a second time when the amplitude of the electrical signal to be measured reaches the second amplitude in order to obtain a second sampling point, which increases the number of the sampling points as sampled, such that the precision of sampling and the accuracy of subsequent signal restoration for the electrical signal may be improved, and it is also possible to obtain other required information, e.g., energy, directly from the information of the sample points as sampled.

Further provided in embodiments of the present application is a signal reconstruction device used for conducting a reconstruction with the first sampling points and the second sampling point sampled by the signal sampling device described in the above embodiments to obtain a reconstructed waveform of the electrical signal to be measured.

Reference may be made to the detailed description of the signal reconstruction method in the foregoing embodiments for the description of the signal reconstruction device, which will not be elaborated herein.

Further provided in the embodiments of the application is a signal processing system, which may comprise the signal sampling device and the signal reconstruction device mentioned above.

The systems, devices, units, etc. described in the above embodiments may be specifically implemented by semiconductor chips, computer chips and/or components, or implemented by products with specific functions. For the convenient purpose, the description of the devices is made respectively of individual units by functions. Apparently, the functions of the individual units may be implemented in one chip or multiple chips when embodying the present application.

Although the method steps described in the above-mentioned embodiments or flowcharts are provided in the disclosure, more or fewer steps may be included in the method with conventional or routine work. Steps, in which there is no necessary causal relationship logically, may be executed in a sequence other than those provided in the embodiments in the disclosure.

The various embodiments in this specification are described in a progressive manner with the same or similar parts to be referred across the various embodiments, while the description of the embodiments focus on differences among different embodiments.

The above-mentioned embodiments are described to facilitate those skilled in the art to understand and practice the application. It is also apparent to those skilled in the art to make various modifications to these embodiments and apply the general principles described herein to other embodiments without creative work. Therefore, the application is not limited to the above-mentioned embodiments, and improvements and modifications may be made by those skilled in the art according to the disclosure without departing from the scope of this application under the spirit of the invention.

**Claims**

1. A signal sampling method, **characterized in that** the signal sampling method comprises:

sampling an electrical signal to be measured using a pre-determined sampling method to obtain a plurality of first sampling points, each of which is represented by a first amplitude and a corresponding first time;
measuring a second amplitude of the electrical signal to be measured, wherein the second amplitude is different from the plurality of the first amplitudes; and

delaying the electrical signal to be measured and using the delayed electrical signal to determine a second time when the amplitude of the electrical signal to be measured reaches the second amplitude in order to obtain a second sampling point, which is represented by the second amplitude and the second time.

2.  The signal sampling method of claim 1, **characterized in that** the step of determining the second time comprises:

measuring a first delay time and a second delay time when the amplitude of the delayed electrical signal reaches any of the first amplitudes and the second amplitude, respectively; calculating a difference between the first delay time and the second delay time; and calculating the second time corresponding to the second amplitude using the difference and the first time corresponding to the first amplitude.

3.  The signal sampling method of claim 1, **characterized in that** the pre-determined sampling method comprises a multi-amplitude threshold sampling method or a time interval sampling method.

4.  The signal sampling method of claim 1, **characterized in that** the electrical signal to be measured comprises one of the following types of pulse signals generated by a PET detector: a sine wave signal, a cosine wave signal, a triangle wave signal, a sawtooth wave signal, a step wave signal, or a square wave signal.

5.  The signal sampling method of claim 1, **characterized in that** said second amplitude comprises a maximum or minimum amplitude of the electrical signal to be measured.

6.  A signal reconstruction method **characterized in that** the signal reconstruction method comprises: conducting a reconstruction with the first sampling points and the second sampling point of the electrical signal to be measured which are sampled by means of the signal sampling method of any of claims 1-5 to obtain a reconstructed waveform of the electrical signal to be measured.

7.  The signal reconstruction method of claim 6, **characterized in that** the steps of conducting a reconstruction with the first sampling points and the second sampling point comprises:

conducting a fitting to the first sampling points and the second sampling point; conducting an interpolation of the first sampling points and the second sampling point; or conducting an interpolation of the first sampling points and the second sampling point, and a fitting to all the sampling points after interpolation, wherein the interpolation comprises a linear interpolation and/or a spline interpolation.

8.  A signal sampling device, **characterized in that** the signal sampling device comprises:

a first sampling unit configured to sample an electrical signal to be measured using a pre-determined sampling method to obtain a plurality of first sampling points, each of which is represented by a first amplitude and a corresponding first time; a measuring unit configured to measure a second amplitude of the electrical signal to be measured, wherein the second amplitude is different from the plurality of the first amplitudes; and a second sampling unit configured to delay the electrical signal to be measured and use the delayed electrical signal to determine a second time when the amplitude of the electrical signal to be measured reaches the second amplitude in order to obtain a second sampling point, which is represented by the second amplitude and the second time.

9.  The signal sampling device of claim 8, **characterized in that** the measuring unit comprises a voltage retaining circuit consisting of a capacitor, a diode, and an inductor, wherein one end of the capacitor and one end of the inductor are connected in parallel and grounded, the other end of the capacitor and one end of the diode are connected in parallel, and the other end of the diode and the other end of the inductor are connected in series.

10. A signal reconstruction device, **characterized in that** the device is configured to conduct a reconstruction with the first sampling points and the second sampling point sampled by the signal sampling device of claim 8 or 9 to obtain a reconstructed waveform of the electrical signal to be measured.

Sampling an electrical signal to be measured using a pre-determined sampling method to obtain a plurality of first sampling points ⟋ S1

Measuring a second amplitude of the electrical signal to be measured ⟋ S2

Delaying the electrical signal to be measured, and using the delayed electrical signal to determine a second time when the amplitude of the electrical signal to be measured reaches the second amplitude in order to obtain a second sampling point ⟋ S3

FIG. 1

FIG. 2

V

Vmax

t

FIG. 3

V

Vmax

t

FIG. 4

First sampling unit 510

Measuring unit 520

Second sampling unit 530

FIG. 5

## INTERNATIONAL SEARCH REPORT

International application No.

**PCT/CN2020/113208**

### A.    CLASSIFICATION OF SUBJECT MATTER

G01T 1/17(2006.01)i

According to International Patent Classification (IPC) or to both national classification and IPC

### B.    FIELDS SEARCHED

Minimum documentation searched (classification system followed by classification symbols)

G01T 1/-;G01R 19/-;H03M 1/12

Documentation searched other than minimum documentation to the extent that such documents are included in the fields searched

Electronic data base consulted during the international search (name of data base and, where practicable, search terms used)

CNABS, CNTXT, VEN, ISI Web of knowledge: 信号, 采样, 延迟, 多阈值, 幅度, 幅值, 精度, 精确, signal, sampl+, delay+, multi+, threthold, amplitude, accura+, precision

### C.    DOCUMENTS CONSIDERED TO BE RELEVANT

| Category* | Citation of document, with indication, where appropriate, of the relevant passages | Relevant to claim No. |
|---|---|---|
| PX | CN 111158039 A (RAYCAN TECHNOLOGY CO., LTD. (SUZHOU)) 15 May 2020 (2020-05-15)<br>claims 1-10, description paragraphs [0042]-[0078], figures 1-5 | 1-10 |
| PX | CN 111103614 A (RAYCAN TECHNOLOGY CO., LTD. (SUZHOU)) 05 May 2020 (2020-05-05)<br>description, paragraphs [0037]-[0060], and figures 2-9 | 1-10 |
| E | CN 211698223 U (RAYCAN TECHNOLOGY CO., LTD. (SUZHOU)) 16 October 2020 (2020-10-16)<br>description, paragraphs [0037]-[0060], and figures 2-9 | 1-3, 5-6, 8-10 |
| Y | CN 102262238 A (RAYCAN TECHNOLOGY CO., LTD. (SUZHOU)) 30 November 2011 (2011-11-30)<br>description, paragraphs [0052]-[0111], and figures 3-4 | 1-10 |
| Y | CN 109444559 A (SUZHOU RAYMEASURE MEDICAL TECHNOLOGY CO., LTD.) 08 March 2019 (2019-03-08)<br>description, paragraphs [0067]-[0077], and figures 2-6 | 1-10 |

☑ Further documents are listed in the continuation of Box C.    ☑ See patent family annex.

| | |
|---|---|
| *    Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **06 November 2020** | **17 November 2020** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2020/113208** |

**C. DOCUMENTS CONSIDERED TO BE RELEVANT**

| Category* | Citation of document, with indication, where appropriate, of the relevant passages | Relevant to claim No. |
| --- | --- | --- |
| Y | CN 103961126 A (RAYCAN TECHNOLOGY CO., LTD. (SUZHOU)) 06 August 2014 (2014-08-06)<br>    description, paragraphs [0049]-[0067], and figures 1-2 | 1-10 |
| Y | CN 105212954 A (RAYCAN TECHNOLOGY CO., LTD. (SUZHOU)) 06 January 2016 (2016-01-06)<br>    description, paragraphs [0053]-[0073], figure 1 | 1-10 |
| Y | H. Kim et al. "A multi-threshold sampling method for TOF-PET signal processing"<br>*Nuclear Instruments and Methods in Physics Research A,*<br>Vol. 602, No. 2, 04 February 2009 (2009-02-04),<br>    section 2, and figure 1-3 | 1-10 |
| A | CN 101710183 A (CHINA INSTITUTE OF ATOMIC ENERGY) 19 May 2010 (2010-05-19)<br>    entire document | 1-10 |
| A | US 2019036759 A1 (ROSHMERE INC) 31 January 2019 (2019-01-31)<br>    entire document | 1-10 |

Form PCT/ISA/210 (second sheet) (January 2015)

**INTERNATIONAL SEARCH REPORT**
Information on patent family members

International application No.

**PCT/CN2020/113208**

| Patent document cited in search report | | | Publication date (day/month/year) | Patent family member(s) | | | Publication date (day/month/year) |
|---|---|---|---|---|---|---|---|
| CN | 111158039 | A | 15 May 2020 | None | | | |
| CN | 111103614 | A | 05 May 2020 | None | | | |
| CN | 211698223 | U | 16 October 2020 | None | | | |
| CN | 102262238 | A | 30 November 2011 | EP | 2700360 | A4 | 17 December 2014 |
| | | | | EP | 2700360 | A1 | 26 February 2014 |
| | | | | CN | 102262238 | B | 23 July 2014 |
| | | | | US | 9772408 | B2 | 26 September 2017 |
| | | | | JP | 5800983 | B2 | 28 October 2015 |
| | | | | JP | 2014516411 | A | 10 July 2014 |
| | | | | US | 2014052414 | A1 | 20 February 2014 |
| | | | | WO | 2012142778 | A1 | 26 October 2012 |
| | | | | BR | 112013026944 | A2 | 10 January 2017 |
| CN | 109444559 | A | 08 March 2019 | WO | 2020082858 | A1 | 30 April 2020 |
| CN | 103961126 | A | 06 August 2014 | EP | 2954838 | A4 | 02 November 2016 |
| | | | | WO | 2014121530 | A1 | 14 August 2014 |
| | | | | US | 9344102 | B2 | 17 May 2016 |
| | | | | CN | 103961126 | B | 06 July 2016 |
| | | | | EP | 2954838 | A1 | 16 December 2015 |
| | | | | US | 2015372689 | A1 | 24 December 2015 |
| CN | 105212954 | A | 06 January 2016 | US | 2018321391 | A1 | 08 November 2018 |
| | | | | JP | 6657398 | B2 | 04 March 2020 |
| | | | | CN | 105212954 | B | 16 March 2018 |
| | | | | EP | 3361288 | A1 | 15 August 2018 |
| | | | | US | 10228470 | B2 | 12 March 2019 |
| | | | | WO | 2017076312 | A1 | 11 May 2017 |
| | | | | EP | 3361288 | A4 | 26 June 2019 |
| | | | | JP | 2018538525 | A | 27 December 2018 |
| CN | 101710183 | A | 19 May 2010 | CN | 101710183 | B | 12 September 2012 |
| US | 2019036759 | A1 | 31 January 2019 | TW | 201911756 | A | 16 March 2019 |
| | | | | WO | 2019023433 | A1 | 31 January 2019 |

Form PCT/ISA/210 (patent family annex) (January 2015)

**REFERENCES CITED IN THE DESCRIPTION**

**Patent documents cited in the description**

- CN 202010000182